# EUROPEAN PATENT SPECIFICATION

(11) **EP 2 907 501 B1**
(45) Date of publication and mention of the grant of the patent: **13.03.2019**
(21) Application number: 13844980.6
(22) Date of filing: 11.10.2013
(51) Int. Cl.: A61K 8/85, A61K 8/02, A61K 8/87, A61Q 17/04, A61K 8/73, A45D 40/30

(54) **SUN CARE COSMETIC**
SONNENPFLEGE KOSMETIK
COSMÉTIQUE DE SOINS SOLEIL

(30) Priority: 12.10.2012 JP 2012227076
(43) Date of publication of application: 19.08.2015
(73) Proprietor: Shiseido Company, Ltd., Chuo-ku Tokyo 104-0061 (JP)
(72) Inventor: KIMURA, Tomoko, Yokohama-shi Kanagawa 224-8558 (JP); KANNO, Naomi, Yokohama-shi Kanagawa 224-8558 (JP); YAMAKI, Satoshi, Yokohama-shi Kanagawa 224-8558 (JP)
(74) Representative: Ter Meer Steinmeister & Partner
(86) International application number: PCT/JP2013/077802
(87) International publication number: WO 2014/058060

(56) References cited:
- EP-A1- 2 082 869
- EP-A1- 2 191 810
- EP-A1- 2 722 169
- WO-A1-2008/129707
- WO-A1-2009/041121
- WO-A1-2013/153678
- WO-A2-2012/087517
- JP-A- H11 228 340
- JP-A- 2013 001 661
- JP-A- 2013 071 906
- JP-A- 2013 071 907
- JP-A- 2013 184 970
- Hiromi Miyazaki et al.: "An ultrathin poly(L-lactic acid) nanosheet as a burn wound dressing for protection against bacterial infection", Wound Repair and Regeneration, vol. 20 19 June 2012 (2012-06-19), pages 573-579, XP002754871, ISSN: 1524-475X Retrieved from the Internet: URL:http://onlinelibrary.wiley.com/doi/10. 1111/j.1524-475X.2012.00811.x/pdf [retrieved on 2016-02-29]
- Toshinori Fujie, Yosuke Okamura and Shinji Takeoka: "Ubiquitous transference of a freestanding polysaccharide nanosheet with the development of a nano-adhesive plaster", Advanced Materials, vol. 19 5 November 2007 (2007-11-05), pages 3549-3553, XP002754872, ISSN: 1521-4095 Retrieved from the Internet: URL:http://onlinelibrary.wiley.com/doi/10. 1002/adma.200700661/pdf [retrieved on 2016-02-29]

## Description

### FIELD OF THE INVENTION

The present invention relates to a sun care cosmetic for use in a method of protecting the skin from UV rays having an excellent secondary adhesion resistance effect.

### BACKGROUND OF THE INVENTION

In makeup cosmetics such as foundation, blusher, eye shadow, and lipstick, a large amount of a coloring agent such as pigment is blended. In order to realize a so-called secondary adhesion resistance effect in which such makeup cosmetics do not adhere to clothes, the blending of a film-forming agent such as silicone-modified polysaccharide compound, described in Patent Literature 1, has been known. However, the realization of the secondary adhesion resistance effect against rubbing has been difficult.

On the other hand, UV rays exert all sorts of harmful effects on the skin. Therefore, to protect the skin, various UV absorbers have been developed and used by blending them into external skin preparations. However, the further improvement of the UV absorption effect has been desired.

Patent Literature 1: Japanese unexamined patent publication No. H10-29910 WO 2012/087517 A2 is directed to cosmetics as solid, water-soluble polymeric films and to such films having plural laminated layers.
WO 2008/129707 A1 relates to cosmetic composition for a facial mask for forming a thin film which comprises a sulfated polysaccharide.

### DISCLOSURE OF THE INVENTION

### PROBLEM TO BE SOLVED BY THE INVENTION

The present invention was made in view of the above-described conventional art. The problem to be solved is to provide a sun care cosmetic for use in a method of protecting the skin from UV rays having an excellent secondary adhesion resistance effect and an excellent UV absorption effect.

### MEANS TO SOLVE THE PROBLEM

The present inventors have diligently studied to solve the above-described problems. As a result, the present inventors have found that a sun care cosmetic for use in a method of protecting the skin from UV rays, having a step of applying a sun care cosmetic on the skin, subsequently a step of pasting a base material film surface of a thin film on the skin, and subsequently a step of removing a support body of the pasted thin film, wherein the thin film consists of a base material film of the thickness of 10 to 500 nm and a support body, has an excellent secondary adhesion resistance effect, thus leading to the completion of the present invention.

That is, the sun care cosmetic of the present invention is defined according to claim 1. Further advantageous embodiments are defined in claims 2 to 6.

A cosmetic kit of the present invention is the kit consists of a non-aqueous emulsion sunscreen and a thin film that consists of a base material film of the thickness of 10 to 500 nm and a support body.

### EFFECT OF THE INVENTION

The present invention can be provided a sun care cosmetic having an excellent secondary adhesion resistance effect and an excellent UV absorption effect.

### BRIEF DESCRIPTION OF THE DRAWINGS

Fig. 1 shows the thin film used in the sun care cosmetic of the present invention.
Fig. 2 is explanatory drawings for the sun care cosmetic of the present invention.
Fig. 3 is explanatory drawings for the sun care cosmetic of the present invention.
Fig. 4 (A-1) is a picture wherein the makeup cosmetic (Reference Example 1) was applied on the skin. Fig. 4 (A-2) is a picture wherein the section on which the cosmetic material was applied (Fig. 4 (A-1)) was rubbed three times with a finger. Fig. 4 (B-1) is a picture wherein a base material film was pasted after the application of the makeup cosmetic (Reference Example 1) on the skin. Fig. 4 (B-2) is a picture wherein the section on which the cosmetic material was applied (Fig. 4 (B-1)) was rubbed three times with a finger.
Fig. 5 (A) are spectra for the sample wherein a polymer emulsion sunscreen (Reference Example 2) was applied and for the sample wherein a base material film was pasted on that.
Fig. 5 (B) are spectra for the sample wherein a water-in-oil sunscreen (Reference Example 3) was applied and for the sample wherein a base material film was pasted on that. Fig. 5 (C) are spectra for the sample wherein a non-aqueous emulsion sunscreen (Reference Example 4) was applied and for the sample wherein a base material film was pasted on that.

### BEST MODE FOR CARRYING OUT THE INVENTION

At first, a thin film used in the sun care cosmetic of the present invention will be explained.

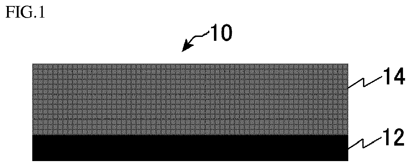

The thin film used in the present invention is shown in Fig. 1.

The thin film 10 consists of a base material film 12 with the film thickness of 10 to 500 nm and a support body 14.

It is necessary that the thickness of base material film is 10 to 500 nm. The thickness of base material film is preferably 50 to 350 nm and especially preferably 100 to 250 nm.

If the thickness of base material film is too thick, an uncomfortable feeling is caused at an application location and the roughness correction effect tends to be poor.

If the thickness of base material film is too thin, the manageability tends to be poor.

The base material film material is not limited in particular; however, it is preferable to use one or more kinds of materials selected from the group consisting of polylactic acid, polyglycolic acid, polycaprolactone, copolymers thereof, and acrylic urethane copolymer.

The preparation method of base material film is not limited in particular so far as the thickness of the present invention can be achieved. However, it is preferable to prepare by a spin-coating method, a (micro) gravure method, or a spray-coating method. In the case of spin coating method, any spin coater can be used; however, it is preferable to carry out preparation at the rotation speed of 2000 to 5000 rpm.

Moreover, sodium hyaluronate is preferably supported on the base material film. In the present invention, the base material film includes the support layer (hyaluronic acid or its derivative) in the case that hyaluronic acid or its derivative is supported.

The thickness of the support layer (hyaluronic acid or its derivative) is not limited in particular; however, it is preferably 50 to 400 nm to produce a uniform product.

As a hyaluronic acid derivative or a derivative thereof, sodium hyaluronate, acetylated hyaluronic acid can be listed.

The supporting of hyaluronic acid or its derivative can be carried out by any method. The preparation of base material film can be carried out by any optional method. For example, the method wherein a solution containing hyaluronic acid or a derivative thereof is dropwise added and dried after the preparation of the base material film by a spin-coating method, a (micro) gravure method, a spray-coating method, etc.; the method wherein a dispersion liquid is dropwise added on the substrate that is used in a spin-coating method, a (micro) gravure method, a spray-coating method, etc. after obtaining the dispersion liquid in which hyaluronic acid or a derivative thereof is dispersed in the base material film material; etc. can be listed, but is not limited in particular.

As a support body, a water-soluble polymer film or a cloth is preferably used. If only the base material film is used, it is too thin, the removal after preparation is difficult, and the manageability is poor. However, a thin film excellent in manageability can be obtained by laminating the support body.

The thickness of the support body is not limited in particular, but it is preferable to be 1 to 500 µm.

The water-soluble polymer film is not limited in particular, but it is preferable to comprise one or more polymer(s) selected from the group consisting of polyvinyl alcohol or a derivative thereof, a polyether or a derivative thereof, polysaccharides, a polymer electrolyte or a salt thereof, and hyaluronic acid or a derivative thereof.

In order to make the handling easy, PET (polyethylene terephthalate) resin film may be laminated as the support body in addition to the water-soluble polymer film when sodium hyaluronate is supported.

The cloth is not limited in particular, but a mesh or a non-woven fabric is preferable.

The mesh means a sheet of network resin, and the examples of materials include PET resin, polyester resin, nylon, and etc. From the standpoint of removability, it is preferable to use PET resin; however, either can be effectively used.

Moreover, examples of non-woven fabrics include nylon, cellulose, polyester fiber, and etc.

In the present invention, the examples of kinds of support bodies and the usage are listed to lead to the state that the base material film is uniformly pasted on the skin; however, the effect of the present patent application can be achieved so far as it can be uniformly pasted on the skin.

The cosmetic method of the present invention is practiced to realize an excellent secondary adhesion resistance effect and realize the improvement of cosmetic long-lasting property.

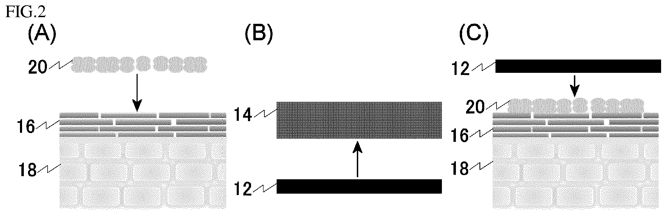

A reference sun care cosmetic has a step of applying a cosmetic material on the skin (Fig. 2 (A)), a step of removing the support body of the thin film (Fig. 2 (B)), and a step of pasting the base material film surface on the skin (Fig. 2 (C)).

At first, as shown in Fig. 2 (A), it is necessary to apply a cosmetic 20 on the skin.

Cosmetic is not limited in particular, but the examples include skin care cosmetic, makeup cosmetic, sun care cosmetic. Among them, sun care cosmetic is preferable.

Sun care cosmetics are cosmetic materials used to protect the skin from UV rays, and a large amount of UV absorber is usually blended. In the present invention, sun care cosmetics are not necessarily for the body only, but the cosmetics used for the face are also included. The examples of sun care cosmetics include sunscreen, beauty essence for daytime use, milky lotion for daytime use, BB cream, and etc.

BB cream is an abbreviation of Blemish Balm cream, and it is a multifunctional cosmetic having the functions of sunscreen and beauty essence as well as the functions of makeup cosmetics such as pre-makeup, foundation, and concealer; therefore, BB cream could be classified to both sun care cosmetics and makeup cosmetics.

As the cosmetic 20 in the present invention, a non-aqueous emulsion sunscreen is especially preferably used. The improvement of the UV absorption effect in the cosmetic method of the present invention can be felt especially by using a non-aqueous emulsion sunscreen.

Makeup cosmetic is not limited in particular, but the examples include foundation, cheek, eye shadow, pre-makeup, lipstick, BB cream, and etc.

The examples of skin care cosmetics include lotion, milky lotion, beauty essence, cream, and etc. When a skin care cosmetic is used, an excellent percutaneous absorption effect of active components can be realized. Therefore, when a skin care cosmetic is used, it is preferable to blend active components such as moisturizer, whitening agent, blood circulation promoter, and plant extracts.

Subsequently, as shown in Fig. 2 (B), it is necessary to remove the support body 14 of the thin film and obtain the base material film 12.

When a water-soluble polymer film is used as the support body, the water-soluble polymer film can be easily removed, for example, by immersing in water.

When cloth is used as the support body, the base material film can pasted on the skin and only the support body can be peeled off by applying water, lotion, etc. on the skin and pasting the base material film side of the thin film on the skin.

As shown in Fig. 2 (C), it is necessary to paste the base material film on the skin.

When hyaluronic acid or its derivative is supported on the base material film, it is preferable that the side supporting hyaluronic acid or its derivative is pasted on the skin.

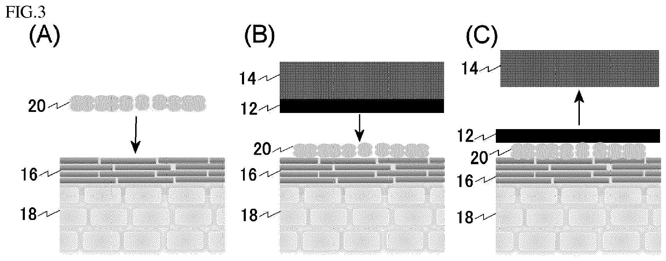

The sun care cosmetic for use in a method of protecting the skin from UV rays of the present invention has a step of applying a sun care cosmetic on the skin (Fig. 3 (A)), a step of pasting the base material film surface of the thin film on the skin (Fig. 3 (B)), and a step of removing the support body of the pasted thin film (Fig. 3 (C)).

These steps can be carried out in the same way as the above reference sun care cosmetic.

In the step shown in Fig. 3 (B), the thin film is pasted on the moderately wetted skin. When cloth is used as the support body, the thin film can be easily pasted on such wetted skin. Thus, in this embodiment, cloth is preferably used as support body.

The base material film pasted by the cosmetic method of the present invention can be peeled off, after use, with water and other cleansers (for example, makeup remover, facial cleanser, etc.).

Thus, the base material film can be removed by even simple water. However, the base material film of the present invention is not removed by normal perspiration only.

### EXAMPLES

Initially, the preparation method of the thin film used in the sun care cosmetic of the present invention will be explained.

### • Preparation method of thin film

All operations were carried out by setting up a spin coater (Opticoat MS-A 150, manufactured by MIKASA Co., Ltd.) inside a clean room (class: 10,000).

Silicon substrate (manufactured by KST World Corp.) was cut into 4 cm × 4 cm, immersed in SPM (H₂SO₄/H₂O₂ = 2.3:1 (v/v)) at 120 °C for 10 minutes, and then washed with ion-exchanged water (specific resistance: 18 MΩcm). This substrate was placed on a spin coater, and the spin coating (4000 rpm, 20 seconds) was carried out by adding dropwise 500 µL of dichloromethane solution of poly-L-lactic acid (hereinafter referred to as PLA) (Mw: 100,000, manufactured by Polysciences Inc., 10 mg/mL) to obtain a poly-L-lactic acid film. The thickness of the obtained poly-L-lactic acid film was measured with an atomic force microscope (manufactured by Keyence Corporation); it was 120 nm.

Subsequently, 10 mg/mL sodium hyaluronate (Biohyalo 12 (manufactured by Shiseido Co., Ltd.), hereinafter referred to as HA) was prepared with 50% and 70% ethanol aqueous solutions. About 1.5 mL of this was dropwise added on the poly-L-lactic acid film and dried (80 °C, 30 minutes), the substrate surface was washed with ion-exchanged water (room temperature, 1 minute), and the surface was dried with nitrogen gas; thus a base material film (a film wherein sodium hyaluronate is supported on the poly-L-lactic acid film; the thickness: 130 nm) was obtained.

In addition, 0.5 mL of polyvinyl alcohol aqueous solution (hereinafter PVA, Mw: 22,000, manufactured by Kanto Chemical Co., Inc., 100 mg/mL) was dropwise added on the surface of the prepared base material film and dried; thus a water-soluble polymer film (PVA film) was formed on the base material film (80 °C, 30 minutes). A thin film was obtained by peeling off, from the silicon substrate, the base material film together with the water-soluble polymer film.

The below-described effects were evaluated by pasting the side of supporting sodium hyaluronate on the skin with the base material film which was obtained by dissolving the water-soluble polymer film by immersing the thin film in water.

At first, the present inventors investigated the skin wherein the base material film of the thin film, obtained by the above-described preparation method, was pasted on the skin to which a makeup cosmetic was applied.

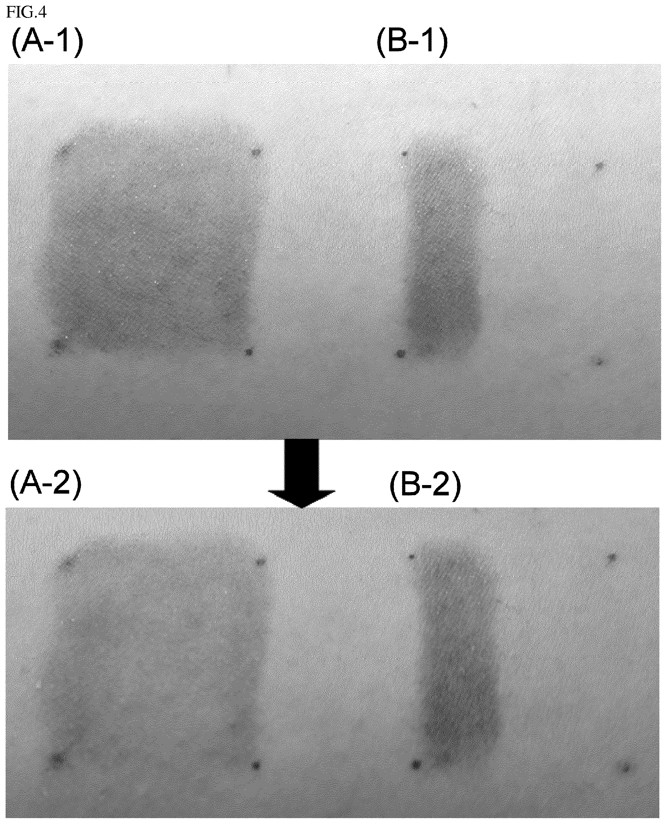

The pictures of the skins (A (control) and B), whose makeup was applied by the below method, are shown in Fig. 4 (A-1) and Fig. 4 (B-1), respectively. Pictures of the removal of eye shadow when the skin was rubbed by a finger three times with the identical force are taken and shown in Fig. 4 (A-2) and Fig. 4 (B-2), respectively.

### • Skin A (control)

Eye shadow of the below-described Reference Example 1 was applied on the non-coated skin with an eye shadow tip.

### • Skin B

Eye shadow of the below-described Reference Example 1 was applied on the non-coated skin with an eye shadow tip. Then, a little water was put on the skin, a base material film was pasted, and it was allowed to stand for 1 minute until the base material film dried.

### Reference Example 1: Eye shadow

| | |
|---|---|
| Talc | 45 mass% |
| Mica | 15 |
| Sericite | 5 |
| Pigment | 15 |
| Pearlescent pigment | 10 |
| Liquid paraffin | 6 |
| Dimethylpolysiloxane | 2 |
| Antioxidant | 2 |
| Preservative | proper quantity |

According to Fig. 4, when a base material film was pasted after the application of a makeup cosmetic, the makeup cosmetic hardly fell off, the cosmetic long-lasting property was improved, and the secondary adhesion resistance effect was excellent.

The present inventors prepared a thin film by the above-described preparation method, and the UV absorption effect of the base material film, when used in combination with a sun care cosmetic, was investigated.

That is, the absorbance in the UV region was measured for the sample wherein each sun care cosmetic of the below-described Reference Examples 2 to 4 was applied on a PMMA plate. Subsequently, a base material film was pasted on each sample, and the absorbance in the UV region was measured for the same section. The measurement was carried out with an automatic recording spectrophotometer "U-3500" (manufactured by Hitachi, Ltd.) at 25 °C.

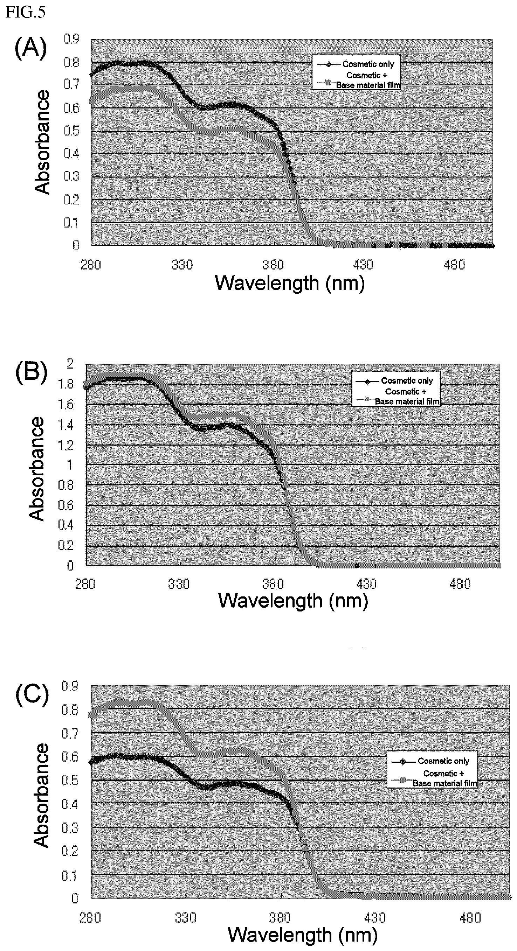

The results for the samples of Reference Examples 2 to 4 are shown in Fig. 5 (A) to Fig. 5 (C), respectively.

### Reference Example 2: Polymer emulsion sunscreen

| | |
|---|---|
| Octyl methoxycinnamate | 8 mass% |
| 4-t-butyl-4'-methoxybenzoylmethane | 2 |
| (Acrylate/alkyl (C10-30) acrylate) crosspolymer | proper quantity |
| Carboxyvinylpolymer | proper quantity |
| Glycerin | 5 |
| Dipropylene glycol | 7 |
| Cetyl 2-ethyl hexanoate | 10 |
| Caustic potash | proper quantity |
| Ion-exchanged water | balance |

### Reference Example 3: Water-in-oil sunscreen

| | |
|---|---|
| Octyl methoxycinnamate | 8 mass% |
| 4-t-butyl-4'-methoxybenzoylmethane | 2 |
| PEG-9 polydimethylsiloxyethyl dimethicone | 1 |
| Methylpolysiloxane network copolymer | 3 |
| Cross-linked silicone/network silicone block copolymer | 3 |
| Dimethylpolysiloxane | 5 |
| Isododecane | 12 |
| Decamethylcyclopentasiloxane | 18 |
| Glyceryl tri-2-ethyl hexanoate | 10 |
| Polybutylene glycol | 1 |
| Trisodium edetate | proper quantity |
| Sodium chloride | proper quantity |
| Ethanol | 5 |
| Glycerin | 5 |
| Ion-exchanged water | balance |

### Reference Example 4: Non-aqueous emulsion sunscreen

| | |
|---|---|
| Octyl methoxycinnamate | 8 mass% |
| 4-t-butyl-4'-methoxybenzoylmethane | 2 |
| Polyoxyethylene(20)/ polyoxypropylene(8) cetyl ether | 3 |
| Glycerin | 5 |
| Dipropylene glycol | 7 |
| Cetyl 2-ethyl hexanoate | 10 |
| Carboxyvinylpolymer | proper quantity |
| Caustic potash | proper quantity |
| Sodium hexamethaphosphate | proper quantity |
| Ion-exchanged water | balance |

According to the results of Fig. 5, the UV absorption rates when a base material film was pasted on the respective cosmetic materials were 84% (Reference Example 2), 105% (Reference Example 3), and 130% (Reference Example 4) with respect to the control (only cosmetic material).

Thus, except for the polymer emulsion sunscreen, the UV absorption effect was found to increase by pasting a base material film on the section where a sun care cosmetic was applied.

In particular, an excellent UV absorption effect was found to be realized by pasting a base material film on the section where a non-aqueous emulsion sunscreen was applied.

Next, the present inventors prepared, by the (micro) gravure method, a thin film containing a base material film of the thickness of 330 nm (poly-L-lactic acid film 250 nm + acetylated hyaluronic acid 80 nm). As the support body, cloth (PET mesh) was used.

The excellent secondary adhesion resistance effect and the UV absorption effect, when a sun care cosmetic was used, could be realized with these thin films similarly to the case that a water-soluble polymer film was the support body.

Furthermore, the present inventors prepared thin films containing base material films (support body: PET mesh, nylon mesh, or non-woven cloth), by the (micro) gravure method, having the thickness other than the above-described thickness (300 nm (poly-L-lactic acid film 200 nm + acetylated hyaluronic acid 100 nm) and 400 nm (poly-L-lactic acid film 200 nm + acetylated hyaluronic acid 200 nm)).

These base material films were pasted on the skin; as a result, all of them were excellent in the secondary adhesion resistance effect and the UV absorption effect when a sun care cosmetic was used.

### DESCRIPTION OF THE NUMERALS

10: Thin film
12: Base material film
14: Support body
16: Stratum corneum
18: Epidermis
20: Cosmetic

## Claims

1. A sun care cosmetic for use in a method of protecting the skin from UV rays having a step of applying a sun care cosmetic on the skin, subsequently a step of pasting a base material film surface of a thin film on the skin, and subsequently a step of removing a support body of the pasted thin film, wherein the thin film consists of a base material film of the thickness of 10 to 500 nm and a support body.

2. The sun care cosmetic for use in a method of protecting the skin from UV rays according to claim 1, wherein the sun care cosmetic is one or more selected from the group consisting of sunscreen, beauty essence for daytime use, milky lotion for daytime use, and BB cream.

3. The sun care cosmetic for use in a method of protecting the skin from UV rays according to any of claim 1 or 2, wherein the sun care cosmetic is a non-aqueous emulsion sunscreen.

4. The sun care cosmetic for use in a method of protecting the skin from UV rays according to any of claims 1 to 3, wherein the base material film comprises one or more kinds of polylactic acid, polyglycolic acid, polycaprolactone, a copolymer thereof, and acrylic urethane copolymer.

5. The sun care cosmetic for use in a method of protecting the skin from UV rays according to any of claims 1 to 4, wherein a film of hyaluronic acid, acetylated hyaluronic acid or sodium hyaluronate is supported on the base material film.

6. The sun care cosmetic for use in a method of protecting the skin from UV rays according to any of claims 1 to 5, wherein the support body is a water-soluble polymer film or a cloth.

7. A cosmetic kit, wherein the kit consists of a non-aqueous emulsion sunscreen and a thin film that consists of a base material film of the thickness of 10 to 500 nm and a support body.

## Patentansprüche

1. Sonnenpflegekosmetikum zur Verwendung bei einem Verfahren zum Schützen der Haut gegenüber UV-Strahlen mit einem Schritt des Auftragens eines Sonnenpflegekosmetikums auf die Haut, nachfolgend einem Schritt des Klebens einer Grundmaterialfilmoberfläche aus einem dünnen Film auf die Haut, und nachfolgend einem Schritt des Entfernens eines Trägerkörpers des geklebten dünnen Films, wobei der dünne Film aus einem Grundmaterialfilm der Dicke von 10 bis 500 nm und einem Trägerkörper besteht.

2. Sonnenpflegekosmetikum zur Verwendung bei einem Verfahren zum Schützen der Haut gegenüber UV-Strahlen nach Anspruch 1, wobei das Sonnenpflegekosmetikum eines oder mehrere ist, gewählt aus der Gruppe, bestehend aus Sonnenschutzmittel, Schönheitsessenz für Tageszeitanwendung, milchige Lotion für Tageszeitanwendung und BB-Creme.

3. Sonnenpflegekosmetikum zur Verwendung bei einem Verfahren zum Schützen der Haut gegenüber UV-Strahlen nach Anspruch 1 oder 2, wobei das Sonnenpflegekosmetikum ein nicht wässriges Emulsion-Sonnenschutzmittel ist.

4. Sonnenpflegekosmetikum zur Verwendung bei einem Verfahren zum Schützen der Haut gegenüber UV-Strahlen nach irgendeinem der Ansprüche 1 bis 3, wobei der Grundmaterialfilm eine oder mehrere Arten von Polymilchsäure, Polyglykoliksäure, Polycaprolacton, einem Copolymer hiervon und einem Acryl-Urethan-Copolymer umfasst.

5. Sonnenpflegekosmetikum zur Verwendung bei einem Verfahren zum Schützen der Haut gegenüber UV-Strahlen nach irgendeinem der Ansprüche 1 bis 4, wobei ein Film aus Hyaluronsäure, acetylierter Hyaluronsäure oder Natriumhyaluronat auf dem Grundmaterialfilm getragen wird.

6. Sonnenpflegekosmetikum zur Verwendung bei einem Verfahren zum Schützen der Haut gegenüber UV-Strahlen nach irgendeinem der Ansprüche 1 bis 5, wobei der Trägerkörper ein wasserlöslicher Polymerfilm oder ein Gewebe ist.

7. Kosmetikkit, wobei das Kit besteht aus einem nicht wässrigen Emulsion-Sonnenschutzmittel und einem dünnen Film, der aus einem Grundmaterialfilm der Dicke 10 bis 500 nm und einem Trägerkörper besteht.

## Revendications

1. Un cosmétique de protection solaire pour usage dans un procédé de protection de la peau des rayons UV comprenant une étape consistant à appliquer un cosmétique de protection solaire sur la peau, ensuite une étape consistant à empâter une surface d'un film de matériau de base d'un film mince sur la peau, et ensuite une étape consistant à retirer le corps de support du film mince empâté, dans lequel le film mince se compose d'un film de matériau de base d'une épaisseur de 10 à 500 nm et d'un corps de support.

2. Le cosmétique de protection solaire pour un usage dans un procédé de protection de la peau des rayons UV selon la revendication 1, dans lequel le cosmétique de protection solaire est un ou plusieurs choisis parmi le groupe constitué par l'écran solaire, l'essence de beauté pour usage durant la journée, la lotion laiteuse pour usage durant la journée, et la crème BB.

3. Le cosmétique de protection solaire pour un usage dans un procédé de protection de la peau des rayons UV selon la revendication 1 ou 2, dans lequel le cosmétique de protection solaire est un écran solaire en émulsion non aqueuse.

4. Le cosmétique de protection solaire pour un usage dans un procédé de protection de la peau des rayons UV selon l'une quelconque des revendications 1 à 3, dans lequel le film de matériau de base comprend un ou plusieurs types d'acide polylactique, d'acide polyglycolique, de polycaprolactone, un copolymère de ceux-ci et un copolymère acrylique d'uréthane.

5. Le cosmétique de protection solaire pour un usage dans un procédé de protection de la peau des rayons UV selon l'une quelconque des revendications 1 à 4, dans lequel un film d'acide hyaluronique, d'acide hyaluronique acétylé ou d'hyaluronate de sodium est supporté sur le film de matériau de base.

6. Le cosmétique de protection solaire pour un usage dans un procédé de protection de la peau des rayons UV selon l'une quelconque des revendications 1 à 5, dans lequel le corps de support et un film de polymère soluble dans de l'eau ou un tissu.

7. Un kit de cosmétique dans lequel le quitte consiste d'un écran solaire en émulsion n'en aqueuse et d'un film mince qui consiste d'un film de matériau de base d'une épaisseur de 10 à 500 nm et d'un corps de support.
